Europäisches Patentamt

European Patent Office

Office européen des brevets

(11) Veröffentlichungsnummer: **0 111 163 B1**

(12) **EUROPÄISCHE PATENTSCHRIFT**

(45) Veröffentlichungstag der Patentschrift:
24.06.87

(21) Anmeldenummer: 83111134.9

(22) Anmeldetag: 08.11.83

(51) Int. Cl.⁴: **A 61 M 15/00**

(54) **Inhaliervorrichtung mit einer Dosiereinrichtung für Inhalate.**

(30) Priorität: 03.12.82 DE 3244836

(43) Veröffentlichungstag der Anmeldung:
20.06.84 Patentblatt 84/25

(45) Bekanntmachung des Hinweises auf die Patenterteilung:
24.06.87 Patentblatt 87/26

(84) Benannte Vertragsstaaten:
AT CH DE FR GB LI NL SE

(56) Entgegenhaltungen:
EP - A - 0 004 039
DE - A - 2 809 255
DE - B - 2 724 175
FR - A - 2 444 504

(73) Patentinhaber: *Bosch-Siemens Hausgeräte GmbH*,
Hochstrasse 17, D-8000 München 80 (DE)

(72) Erfinder: **Bernitz, Franz, Ringstrasse 18, D-8011 Anzing (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Vorliegende Erfindung bezieht sich auf eine Inhaliervorrichtung mit einer Dosiervorrichtung für Inhalate unter Verwendung eines hermetisch geschlossenen Inhalatsbehälters, der unter elastischem Druck stehende Inhalatsflüssigkeit enthält und der mit einer in Richtung auf den Bereich eines Zerstäuberelements gerichteten Düse ausgestattet ist, durch die beim Öffnen eines dieser Düse zugeordneten Ventils die Inhalatsflüssigkeit vom Vorratsbehälter zum Zerstäuberelement gelangt.

Bei bekannten Inhalationsgeräten, die z.B. mit einer medizinischen Wirkstoff-Flüssigkeit angefüllt sind und die zur Behandlung der Atemwege, beispielsweise bei Asthma-Erkrankten, dienen, erfolgt die Dosierung des flüssigen Wirkstoffes auf mechanischem Wege über einen auf dem Pumpenprinzip beruhenden Fördermechanismus, mittels welchem eine vorbestimmte Portion der Flüssigkeit zu einem Zerstäuberelement, z.B. zu einer Zerstäuberdüse gebracht werden kann (DE-C 2 854 841). Insbesondere bei Inhalationsgeräten für Asthma-Kranke, deren Flüssigkeitsbehälter mehrere Inhalationsportionen enthalten, ist es von ausserordentlicher Wichtigkeit, dass bei jedem Inhalationsvorgang dem Asthma-Kranken eine vorbestimmte, exakt dosierte Inhalatmenge zugeführt wird. Schon relativ geringe Abweichungen von dieser Sollmenge führen entweder zur völligen Wirkungslosigkeit des Wirkstoffes oder aber zu schwerwiegenden Schädigungen der Atemwege der behandelten Person. Bekannte Inhalationsgeräte der vorgenannten Art, die nach dem Kolben-Saugpumpen-Prinzip arbeiten besitzen zur Erlangung einer exakten Dosierung der Inhalatportionen eine konstruktiv sehr aufwendige Bauweise, wobei zu befürchten ist, dass bei nicht exakter Einhaltung der sehr engen Konstruktionstoleranzen eine genaue gleichbleibende Dosierung des Inhalats nicht mehr gewährleistet ist.

Um diesem Mangel zu begegnen, ist eine Dosiereinrichtung für Inhalate bekannt geworden (DE-B 2 934 732), bei der als Pumpeinrichtung eine mit der Inhalatsflüssigkeit in Verbindung stehende Zahnradpumpe vorgesehen ist, deren mit dem Inhalatbehälter luftdicht verbundenes Pumpengehäuse einen zum Zerstäuberelement führenden Austrittskanal aufweist und die durch ein Betätigungsorgan entsprechend der gewünschten Dosierung betätigbar ist. Soll eine derartige Vorrichtung die Forderungen an eine erforderliche Dosiergenauigkeit des Inhalats erbringen, so ist deren Herstellungsaufwand erheblich. Der vorliegenden Erfindung liegt nunmehr die Aufgabe zugrunde, eine Dosiereinrichtung für Inhalate der eingangs genannten Art so auszugestalten, dass bei einfacher und raumsparender konstruktiver Ausgestaltung ein Höchstmass an Sicherheit in bezug auf die exakte Dosierung der Inhalats-Portionen gewährleistet ist. Es ist zu berücksichtigen, dass mechanische Vorstellungstoleranz-Ungenauigkeiten sich möglichst kaum auf die Inhalat-Portionierung auswirken und dass eine Möglichkeit zum Ausgleich dieser Toleranz-Ungenauigkeiten auf einfache Art und Weise gegeben ist. Insbesonders ist der Umstand zu berücksichtigen, dass die Flüssigkeitsmengen des Inhalats im Vorratsbehälter oder im Leitungssystem von schädlichen Einflüssen, wie z.B. Umgebungsluft oder Bakterien geschützt werden sollen.

Eine Inhaliervorrichtung mit einer Dosiereinrichtung für Inhalate, die diesen Forderungen in vollem Umfang entspricht, ist erfindungsgemäss dadurch gekennzeichnet, dass das Ventil ein durch eine Zeitsteueranordnung steuerbares Ventil ist und dass der auf die Inhalatsflüssigkeit wirkende elastische Druck ausschliesslicher Förderdruck für die Inhalatsflüssigkeit ist.

Eine nach diesen erfindungsgemässen Merkmalen ausgestattete Inhaliervorrichtung mit einer Dosiereinrichtung für Inhalate zeichnet sich dadurch aus, dass für die Dosierung des Inhalats bei der Zuführung an das Zerstäuberelement neben dem auf das Inhalat wirkenden elastischen Druck die Austrittsdüse und die Zeitsteueranordnung ausschlaggebend ist. Düsen sind mit hoher Präzision einfach herstellbar. Der auf das Inhalat wirkende elastische Druck wird durch den Hersteller der Inhalatfüllungen mit der nötigen Genauigkeit vorgegeben. Durch die Verwendung einer auf das Ventil wirkenden Zeitsteueranordnung ist darüber hinaus die Möglichkeit für die Einstellbarkeit einer Inhalat-Dosiermenge gegeben, wobei Herstellungs-Toleranzen durch Veränderung des Zeitfaktors einfach korrigierbar sind. Dies ist insbesondere dann der Fall, wenn eine elektronische Zeitsteueranordnung vorgesehen ist. Bevorzugterweise wirkt diese auf ein im Bereich der Düse des Inhalationsbehälters angeordnetes elektromagnetisches Ventil.

Nach einer bevorzugten Weiterbildung ist die erfindungsgemässe Inhaliervorrichtung dadurch gekennzeichnet, dass die Zeitsteueranordnung für das Ventil zusätzlich der Aktivierung des Zerstäuberelements zugeordnet ist. Bei einer dermassen ausgestalteten Inhaliervorrichtung wird durch eine einzige Betätigung eines Auslöseorgans an der Inhaliervorrichtung das dem Zerstäuberelement durch die Dosiervorrichtung zugeführte Inhalat sofort zerstäubt. Die Ansteuer-Vorrichtung ist sicherheitshalber zweckmässig so ausgestaltet, dass eine wiederholte dosierte Zuführung von Inhalatflüssigkeit an das Zerstäuberelement erst dann wieder möglich ist, wenn der Betätigungsmechanismus der Inhaliervorrichtung vorher wieder freigegeben wurde.

Nach einer bevorzugten Ausgestaltung ist die erfindungsgemässe Inhaliervorrichtung dadurch gekennzeichnet, dass das elektromagnetische Ventil gebildet ist durch einen im Inhalatbehälter angeordneten Magnetanker mit steuerbarer Schliessventilfunktion und durch eine in der Inhaliereinrichtung angeordnete Elektromagnetspule für die Magnetanker. Nach einer anderen bevorzugten Ausgestaltung ist die erfindungsgemässe Inhaliervorrichtung dadurch gekennzeichnet,

dass das elektromagnetische Ventil gebildet ist durch einen Magnetanker und eine elektrisch mit der Zeitsteuerung der Inhaliervorrichtung kontaktierbaren Elektromagnetspule im Inhalatbehälter. Ist die Elektromagnetspule Bestandteil der Inhaliereinrichtung, so kann die Zeitsteuerung unmittelbar für die elektromagnetische Charakteristik dieser Spule einjustiert sein.

Im Rahmen der Erfindung sind weitere bevorzugte Ausgestaltungen der Inhaliervorrichtung wahlweise realisierbar. So ist es beispielsweise möglich, im Inhalatbehälter einen durch das – oder federdruckbeaufschlagten – auf die Inhalationsflüssigkeit wirkenden Kolben anzuordnen. Dieser Kolben, welcher auch durch eine Membrane ersetzt sein kann, dient zur möglichst vollkommenen Trennung des Inhalats von Umwelteinflüssen. Andererseits bietet es sich an, dass der Inhalatsbehälter mit seiner Düse durch das Zerstäuberelement hindurch in den Bereich des Zentrums, dessen Zerstäuberfläche geführt ist, insbesondere dann, wenn das Zerstäuberelement bevorzugterweise ein Biegeschwinger ist. Damit wird eine Zuführung des Inhalats an das Zerstäuberelement in unmittelbarer Nähe durchgeführt, ohne dass diese Zuführung die Zerstäubung des Inhalats nachteilig beeinflussen könnte.

Mit Merkmalen der Erfindung ausgestattete Ausführungsbeispiele sind anhand der Zeichnung im folgenden näher beschrieben. Es zeigen:

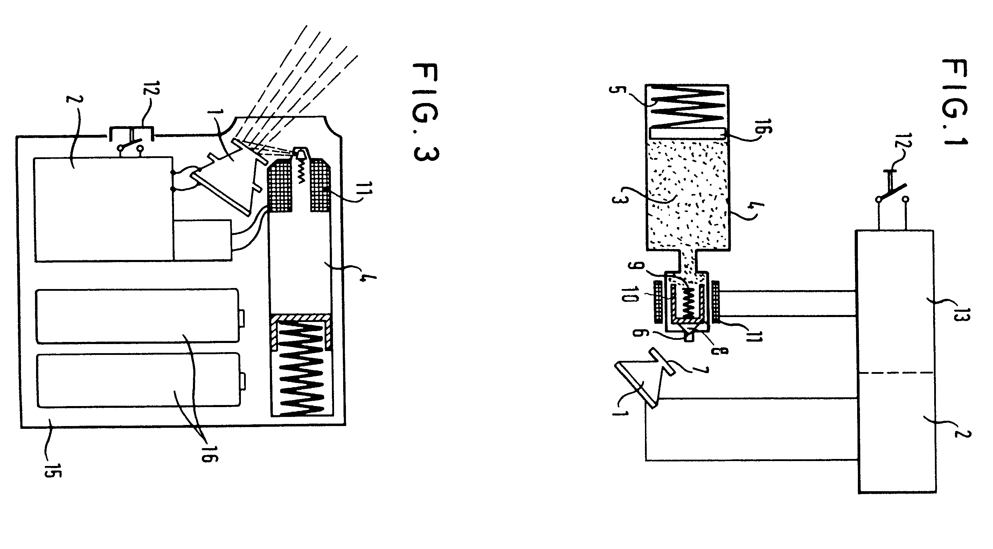

Fig. 1 eine schematische Darstellung der Inhaliervorrichtung mit einer Dosiereinrichtung,

Fig. 2 eine Einzelheit im Bereich des Zerstäuberelementes und der diesem nahen Dosiervorrichtung,

Fig. 3 eine schematische Darstellung der Anordnung der Bauteile in einem handlichen Inhalator und

Fig. 4 eine weitere Zuordnung der Dosiereinrichtung zum Zerstäuberelement.

Als Zerstäuberelement 1 ist im vorliegenden Ausführungsbeispiel ein Piezo-Biegeschwingersystem angeordnet, welches durch eine entsprechende, ebenfalls bekannte elektronische Schaltungsanordnung ansteuerbar ist. Die zu zerstäubende Inhalatflüssigkeit 3 ist von einem Inhalatbehälter 4 hermetisch geschützt gegen Umwelteinflüsse. Ein Federelement 5 übt über einen Kolben 16 einen elastischen Druck auf die Inhalatsflüssigkeit 3 aus. Eine Düse 6 des Inhalatbehälters 4 ist in Richtung auf die Zerstäuberfläche 7 des Zerstäuberelements 1 ausgerichtet und durch ein Ventil 8 über eine Druckfeder 9 verschlossen. Dieses Ventil 8 beinhaltet zumindest einen Teilbereich 10 aus ferromagnetischem Material, welcher die Aufgabe eines Tauchankermagneten übernimmt. Zur Ansteuerung dieses Tauchankermagneten 10 dient eine elektromagnetische Spule 11.

Bei Betätigung eines Tastschalters 12 wird innerhalb der elektronischen Schaltungsanordnung 2 eine Zeitsteueranordnung ausgelöst. In Abhängigkeit dieses Auslösekriteriums wird die elektromagnetische Spule 11 für eine vorgegebene Zeitspanne erregt, so dass über die Tauchankerfunktion das Ventil 8 die Düse 6 für diese Zeitspanne freigibt und die unter Druck stehende Inhalatflüssigkeit 3 in Richtung auf die Zerstäuberfläche 7 des Zerstäuberelements 1 portioniert gespritzt wird. Gleichzeitig wird über die elektronische Schaltungsanordnung 2 das Zerstäuberelement 1 erregt, so dass die auf die Zerstäuberfläche 7 gelangte Inhalatmenge atmungsgerecht zerstäubt wird.

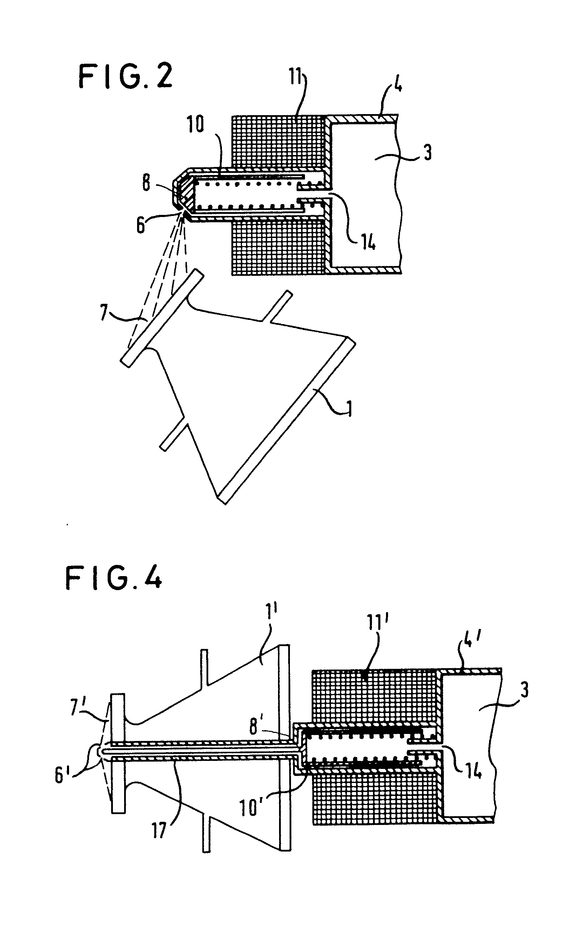

Aus Fig. 2 ist eine Einzelheit im Bereich der Düse 6 des Inhalatbehälters 4 und des Zerstäuberelements ersichtlich. Die Düse 6 ist so ausgestaltet und angeordnet, dass die Zerstäuberfläche durch das auftreffende Inhalat möglichst gleichmässig so benetzt wird, dass eine gute Zerstäubungswirkung erreicht wird. Um den bei geöffneter Düse 6 anstehenden Druck der Inhalatflüssigkeit vom anstehenden Druck im Inhalatbehälter 4 möglichst zu entkoppeln, d.h. um diesen dynamischen Druck möglichst konstant auszulegen, ist zwischen der Ventilkammer und dem eigentlichen Vorratsraum für die Inhalatflüssigkeit 3 innerhalb des Inhalatbehälters 4 eine Reduzierdüse 14 angeordnet.

Die Fig. 3 zeigt die Inhaliervorrichtung mit einem Gehäuse 15, welches den auswechselbaren Inhalatbehälter 4, des Zerstäuberelements 1, die Ansteuerelektronik 2 und Stromquellen in Form von Batterien 16 umgibt.

Bei der Ausführungsform gemäss Fig. 4 ist der Inhalatsbehälter 4' auf der Rückseite des Zerstäuberelements 1 angeordnet und mit einem Kanal 17 ausgestattet, welcher durch das Zerstäuberelement 1' mittig hindurchragt und im mittigen Bereich vor der Zerstäuberfläche 7' Austrittsdüsen 6' für die Inhalatflüssigkeit 3 aufweist. Damit wird das portionsweise ausgestossene Inhalat äusserst nahe durch die Düsen 6' an die Zerstäuberfläche 7' des Zerstäuberelements 1 herangebracht, ohne dass das Zuführungsorgan 17 für die Inhalatflüssigkeit den Zerstäubungsbereich störend beeinflussen könnte.

**Patentansprüche**

1. Inhaliervorrichtung mit einer Dosiervorrichtung für Inhalate unter Verwendung eines hermetisch geschlossenen Inhalatbehälters (4, 4'), der eine unter elastischem Druck stehende Inhalatsflüssigkeit (3) enthält und der mit einer in Richtung auf den Bereich eines Zerstäuberelements (1, 1') gerichteten Düse (6', 6') ausgestattet ist, durch die beim Öffnen eines dieser Düse (6, 6') zugeordneten Ventils (8, 8') die Inhalatsflüssigkeit (3) vom Vorratsbehälter (4, 4') zum Zerstäuberelement (1, 1') gelangt, dadurch gekennzeichnet, dass das Ventil (8, 8') ein durch eine Zeitsteueranordnung (13) steuerbares Ventil ist und dass der auf die Inhalatsflüssigkeit (3) wirkende elastische Druck ausschliesslicher Förderdruck für die Inhalatsflüssigkeit (3) ist.

2. Inhaliervorrichtung nach Anspruch 1, dadurch gekennzeichnet, dass als Zeitsteueranordnung (13) ein elektronisches Zeitschaltglied vorgesehen ist.

3. Inhaliervorrichtung nach einem der Ansprüche 1 oder 2, dadurch gekennzeichnet, dass ein elektromagnetisches Ventil (8) im Bereich der Düse (6) des Inhalatbehälters (3) angeordnet ist.

4. Inhaliervorrichtung nach Anspruch 3, dadurch gekennzeichnet, dass das elektromagnetische Ventil (8, 8′) gebildet ist durch einen im Inhalatsbehälter (4, 4′) angeordneten Magnetanker (10, 10′) mit steuerbarer Schliessventilfunktion und durch eine in der Inhaliereinrichtung angeordnete Elektromagnetspule (11, 11′) für diesen Magnetanker (10, 10′).

5. Inhaliervorrichtung nach Anspruch 3, dadurch gekennzeichnet, dass das elektromagnetische Ventil (8, 8′) gebildet ist durch einen Magnetanker (10, 10′) und eine elektrisch mit der Zeitsteuerung der Inhaliervorrichtung kontaktierbare Elektromagnetspule (11, 11′) im Inhalatsbehälter (4, 4′).

6. Inhaliervorrichtung nach einem der Ansprüche 1 bis 5, dadurch gekennzeichnet, dass die Zeitsteueranordnung (13) für das Ventil (8, 8′) zusätzlich der Aktivierung des Zerstäuberelements (1, 1′) zugeordnet ist.

7. Inhaliervorrichtung nach einem der Ansprüche 1 bis 6, dadurch gekennzeichnet, dass im Inhalatbehälter ein durch Gas- oder Federdruck (5) beaufschlagter, auf die Inhalatsflüssigkeit wirkender Kolben (16) angeordnet ist.

8. Inhaliervorrichtung nach einem der Ansprüche 1 bis 7, dadurch gekennzeichnet, dass zwischen einem Strömungsventil (14) und der Düse (6′) eine Zwischenkammer (17) gebildet ist.

9. Inhaliervorrichtung nach einem der Ansprüche 1 bis 8, dadurch gekennzeichnet, dass der Inhalatsbehälter (3′) mit seiner Düse (6′) durch das Zerstäuberelement (1′) in den Bereich des Zentrums dessen Zerstäuberfläche (7′) geführt ist.

10. Inhaliervorrichtung nach einem der Ansprüche 1 bis 9, dadurch gekennzeichnet, dass das Zerstäuberelement (1, 1′) ein Biegeschwingungssystem ist.

**Claims**

1. Inhalator device comprising a dosage mechanism for inhalates using an inhalator tank (4, 4′) closed hermetically containing an inhalator fluid (3) exposed pressure, which tank is provided with a nozzle (6, 6′) directed to the area of a diffuser element (1, 1′), through which nozzle by opening of a valve attached to the nozzle the inhalator fluid (3) comes from the tank (4, 4′) to the diffuser element (1, 1′), characterized in that the valve (8, 8′) is a valve controlable by a time control arrangement (13) and in that the elastical pressure acting to the inhalator fluid (3) is the exclusive conveying pressure for the inhalator fluid.

2. Inhalator device according to claim 1, characterized in that a time control arrangement (13) is provided an electronic timing switch element.

3. Inhalator device according to one of claims 1 or 2, characterized in that an electromagnetic valve (8) is arranged in the area of the valve (6) of the inhalator tank (4).

4. Inhalator device according to claim 3, characterized in that the electromagnetic valve (8, 8′) is constituted by a magnetic anchor (10, 10′) with controlable closing function and by an electromagnetic coil (11, 11′) for said magnet anchor arranged inside of the inhalator device.

5. Inhalator device according to claim 3, characterized in that the electromagnetic valve (8, 8′) is provided by a magnet anchor (10, 10′) and an electromagnetic coil electrically contactable with the time controlling of the inhalator device inside of the inhalator tank.

6. Inhalator device according to one of the claims 1 to 5, characterized in that the time control arrangement (13) for the valve (8, 8′) is appointed additionally to the activation element of the diffuser element (1, 1′).

7. Inhalator device according to one of the claims 1 to 6, characterized in that inside of the inhalator tank a piston (16) is arranged, operating by pressure (5) of gas or a spring, acting on the inhalator fluid.

8. Inhalator device according to one of the claims 1 to 7, characterized in that between a stream valve (14) and the valve (6) is formed an intermediate chamber.

9. Inhalator device according to one of the claims 1 to 8, characterized in that the inhalator tank (3′) with its valve (6′) is directed through the diffuser element (1′) into the area of the middle of the diffuser field (7).

10. Inhalator device according to one of the claims 1 to 9, characterized in that the diffuser element (1, 1′) is a bending oszillation system.

**Revendications**

1. Appareil d′inhalation comprenant un dispositif de dosage des produits à inhaler, en utilisant un récipient à produit à inhaler (4, 4′) fermé d′une manière hermétique, qui contient du liquide à inhaler (3) soumis à une pression élastique et qui est muni d′une buse (6, 6′) dirigée en direction de la région d′un élément de pulvérisation (1, 1′) et par laquelle, lors de l′ouverture d′une soupape (8, 8′) associée à cette buse (6, 6′), le liquide à inhaler (3) passe du récipient réservoir (4, 4′) à l′élément de pulvérisation (1, 1′), caractérisé en ce que la vanne (8, 8′) est une vanne qui peut être commandée par un dispositif de commande en fonction du temps (13) et en ce que la pression élastique agissant sur le liquide à inhaler (3) est exclusivement la pression de refoulement du liquide à inhaler (3).

2. Appareil d′inhalation suivant la revendication 1, caractérisé en ce qu′il est prévu, comme dispositif de commande en fonction du temps (13), un élément électronique de minuterie.

3. Appareil d′inhalation suivant l′une des revendications 1 ou 2, caractérisé en ce qu′une électrovanne (8) est disposée dans la région de la buse (6) du récipient à produit à inhaler (4, 4′).

4. Appareil d'inhalation suivant la revendication 3, caractérisé en ce que l'électrovanne (8, 8′) est constituée de l'armature d'un aimant (10, 10′), disposé dans le récipient à produit à inhaler (4, 4′) et ayant une fonction de fermeture de la vanne qui peut être commandée, et d'une bobine d'électroaimant (11, 11′), disposée dans l'appareil à inhaler et destinée à cette armature d'aimant (10, 10′).

5. Appareil d'inhalation suivant la revendication 3, caractérisé en ce que l'électrovanne (8, 8′) est constituée d'une armature d'aimant (10, 10′) et d'une bobine d'électroaimant (11, 11′), qui est dans le récipient à produit à inhaler (4, 4′) et qui peut être mise en contact électrique avec le dispositif de commande en fonction du temps de l'appareil d'inhalation.

6. Appareil d'inhalation suivant l'une des revendications 1 à 5, caractérisé en ce que le dispositif de commande en fonction du temps (13) de la vanne (8, 8′) est associé, en outre, à la mise en fonctionnement de l'élément de pulvérisation (1, 1′).

7. Appareil d'inhalation suivant l'une des revendications 1 à 6, caractérisé en ce que, dans le récipient à produit à inhaler, est disposé un piston (16) chargé par la pression d'un gaz ou par la pression d'un ressort (5) et agissant sur le liquide à inhaler.

8. Appareil d'inhalation suivant l'une des revendications 1 à 7, caractérisé en ce qu'une chambre intermédiaire (17) est formée entre une vanne de régulation du débit (14) et la buse (6′).

9. Appareil d'inhalation suivant l'une des revendications 1 à 8, caractérisé en ce que le récipient à produit à inhaler (3′) est guidé par sa buse (6′) dans l'élément de pulvérisation (1′) dans la région du centre de la surface de pulvérisation (7′) de celui-ci.

10. Appareil d'inhalation suivant l'une des revendications 1 à 9, caractérisé en ce que l'élément de pulvérisation (1, 1′) est un système oscillant en flexion.

# FIG.1

# FIG.3

# FIG.2

# FIG.4